(19) Europäisches Patentamt
European Patent Office
Office européen des brevets

(11) Publication number: 0 466 096 A1

(12) EUROPEAN PATENT APPLICATION

(21) Application number: 91111404.9

(22) Date of filing: 09.07.91

(51) Int. Cl.5: C07C 317/22, C07C 317/32, B41M 5/30

(30) Priority: 12.07.90 JP 184727/90
13.03.91 JP 47157/91

(43) Date of publication of application:
15.01.92 Bulletin 92/03

(84) Designated Contracting States:
BE DE FR GB IT SE

(71) Applicant: JUJO PAPER CO., LTD.
No. 4-1, Oji 1-chome
Kita-ku Tokyo(JP)

Applicant: Yoshitomi Pharmaceutical Industries, Ltd.
35 Hiranomachi 3-chome Higashi-ku
Osaka-shi Osaka 541(JP)

(72) Inventor: Minami, Toshiaki
Nishigahara shataku 107, 8-2, Nishigahara 2-chome
Kita-ku, Tokyo(JP)
Inventor: Ohashi, Reiji
Nishigahara shataku 107, 8-2, Nishigahara 2-chome
Kita-ku, Tokyo(JP)
Inventor: Umeda, Hiroaki
Oji-5-chome shataku 805 21-3, Oji 5-chome
Kita-ku, Tokyo(JP)
Inventor: Kinishi, Ryoichi
345, Naoe, Yoshitomi-cho
Chikujo-gun, Fukuoka-ken(JP)
Inventor: Shimada, Akira
42-1, Oaza-Kanate
Nakatsu-shi, Oita-ken(JP)

(74) Representative: Kraus, Walter, Dr. et al
Patentanwälte Kraus, Weisert & Partner
Thomas-Wimmer-Ring 15
W-8000 München 22(DE)

(54) **Derivatives of 4-hydroxyphenylsulfone.**

(57) 4-Hydroxydiphenylsulfone derivatives represented by formula (I)

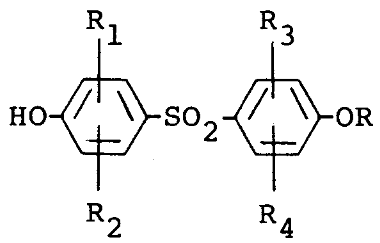

(I)

wherein
$R_1$, $R_2$, $R_3$ and $R_4$, independently from one another, each denote a hydrogen atom, an alkyl group, a substituted or unsubstituted phenyl group or a cycloalkyl group, provided at least one of $R_1$, $R_2$, $R_3$ and $R_4$ is other than a hydrogen atom, and
R denotes an alkyl group, an alkenyl group, a substituted or unsubstituted phenyl group, a substituted or unsubstituted cycloalkyl group or a substituted or unsubstituted phenylalkyl group.

Said compounds are useful as a developer in a heat sensitive color formation layer of a heat sensitive recording material.

EP 0 466 096 A1

This invention relates to novel phenolic compounds. More specifically, this invention relates to novel 4-hydroxydiphenylsulfone derivatives and use of said derivatives as a developer in a heat sensitive recording material.

In general, a heat sensitive recording sheet is prepared by grinding a colorless or light-colored basic leuco dye and an organic developer such as a phenolic compound, or the like into fine particles, mixing them, further adding a binder, a filler, a sensitivity improving agent, a lubricant and other aids to the mixture, and coating the resultant coating solution on a support sheet such as paper, a synthetic paper, a plastic film, or the like. The color formation recording can be provided by the instantaneous chemical reaction upon heating with a heat sensitive head, a hot stamp, a laser beam, etc.

Said heat sensitive recording sheet has been applied to the wide-ranging fields such as a measuring recorder, a terminal printer of a computer, a facsimile, a ticket vending machine, a bar code label, and the like. According to a variety of these recording devices and their higher performance, higher qualities are required of the heat sensitive recording sheet. For instance, as a recording speed becomes higher, it is required that a vivid color formation image is obtained in a high concentration even with a minimal heat energy. Meanwhile, a heat sensitive recording sheet of excellent preservability having light resistance, weatherability, oil resistance and water resistance is demanded.

To meet these requests, technologies using varied 4-hydroxydiphenylsulfone derivatives as a developer have been disclosed before (see U.S. Patent 4,453,744, EP-A-67793, U.S. Patent 4,446,209, GB-B-2,112,156, Japanese Laid-open Patent Application No. 20,493/1983, U.S. Patent 4,616,239, GB-B-2,142,630, and Japanese Laid-open Patent Application Nos. 13,852/1985 and 119,095/1987).

On the other hand, the heat sensitive recording sheets have been widespread, and after the heat sensitive recording, writing with a felt pen of an oil ink has been conducted in many cases. The oil ink, however, contains various organic solvents. For example, when a black-forming heat sensitive recording sheet obtained by using the 4-hydroxydiphenylsulfone derivatives as a developer is written with an oil red ink, the black color inherent in the heat sensitive recording sheet and the red color of the red ink are mixed owing to the solvent of the ink, so that writing with a pure red color cannot be provided. This is the same with inks of the other colors.

A principal object of this invention is to provide 4-hydroxydiphenylsulfone derivatives suited as a developer in a heat sensitive recording sheet, said derivatives being free from the aforesaid defects.

Another object of this invention is to provide a heat sensitive recording sheet having high sensitivity and using such 4-hydroxydiphenylsulfone derivatives, said sheet being capable of writing with a vivid color using an oil ink.

The other objects and advantages of this invention will be clarified by the following detailed description.

In accordance with this invention, there are provided 4-hydroxydiphenylsulfone derivatives represented by formula (I)

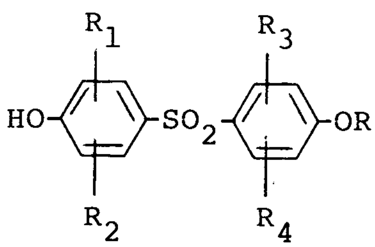

(I)

wherein

$R_1$, $R_2$, $R_3$ and $R_4$, independently from one another, each denote a hydrogen atom, an alkyl group, a substituted or unsubstituted phenyl group or a cycloalkyl group, provided at least one of $R_1$, $R_2$, $R_3$ and $R_4$ is other than a hydrogen atom, and

R denotes an alkyl group, an alkenyl group, a substituted or unsubstituted phenyl group, a substituted or unsubstituted cycloalkyl group or a substituted or unsubstituted phenylalkyl group.

The "alkyl group" here referred to may be either linear or branched. Examples of the alkyl group include methyl, ethyl, propyl, isopropyl, butyl, isobutyl, sec-butyl, tert-butyl, pentyl, isoamyl, hexyl, heptyl, octyl, 2-ethylhexyl and decyl. In this case, the alkyl group has not more than 10 carbon atoms, and a lower alkyl group is preferable.

The "lower" here referred to means that a group or a compound to which this word is applied has not more than 6 carbon atoms, preferably not more than 4 carbon atoms.

The "alkenyl group" may be either linear or branched. Examples of the alkenyl group include vinyl, allyl, propenyl, butenyl, isobutenyl, pentenyl and hexenyl. Of these, a lower alkenyl group, above all, a $C_2$-$C_3$ alkenyl group is preferable.

The "cycloalkyl group" can have 3 to 10 carbon atoms, preferably, 5 to 8 carbon atoms. Examples of the cycloalkyl group include cyclopropyl, cyclopentyl, cyclohexyl and cyclooctyl.

The "phenylalkyl group" is a phenyl-substituted alkyl group in which the alkyl moiety has the aforesaid meaning. Examples of the phenylalkyl group include benzyl, phenetyl, gamma-phenylpropyl, delta-phenylbutyl, epsilon-phenylpentyl and zeta-phenylhexyl. Of these, benzyl is preferable.

Examples of the substituent present in the phenyl ring or the cycloalkyl group in the expressions "substituted or unsubstituted phenyl group", "substituted or unsubstituted cycloalkyl group" and "substituted or unsubstituted phenylalkyl group" include a halogen atom such as chlorine, bromine or fluorine, especially chlorine; a lower alkyl group; a lower alkoxy group such as methoxy or ethoxy; a nitro group; an amino group; and the like. The phenyl ring or the cycloalkyl ring can have 2 or more of these substituents, but 1 substituent is preferable. Examples of the substituted phenyl group include p-chlorophenyl, p-bromophenyl, p-methylphenyl, m-methylphenyl, p-ethylphenyl, p-methoxyphenyl, m-ethoxyphenyl and p-nitrophenyl. Examples of the substituted cycloalkyl group include 4-chlorocyclohexyl, 2-bromohexyl, 4-methylcyclohexyl, 4-methoxycyclohexyl, 3-nitrocyclohexyl and 2-aminocyclohexyl. Examples of the substituted phenylalkyl group include p-bromobenzyl, p-methylbenzyl, m-methylbenzyl, p-ethylphenetyl, p-methoxyphenetyl, m-ethoxyphenetyl and p-nitrophenetyl.

In formula (I), preferably, $R_1$, $R_2$, $R_3$ and $R_4$, independently from one another, are each a hydrogen atom, a lower alkyl group, a phenyl group that may optionally be substituted by a halogen atom, or a cyclohexyl group, provided at least one of $R_1$, $R_2$, $R_3$ and $R_4$ is other than the hydrogen atom;

more preferably, $R_1$ and $R_3$, independently from each other, are each a hydrogen atom, a lower alkyl group, or a phenyl group that may optionally be substituted by chlorine, or a cyclohexyl group, provided $R_1$ and $R_3$ are not hydrogen atoms at the same time, and $R_2$ and $R_4$ are each a hydrogen atom; and

most preferably, $R_1$ and $R_3$, independently from each other, are each a hydrogen atom or a methyl group, provided $R_1$ and $R_3$ are not hydrogen atoms at the same time, and $R_2$ and $R_4$ are each a hydrogen atom.

On the other hand, preferably, R is a lower alkyl group, a $C_2$-$C_3$ alkenyl group, a phenyl group that may optionally be substituted by halogen, lower alkyl or lower alkoxy, a cyclohexyl group that may optionally be substituted by halogen, or a phenyl-lower alkyl group that may optionally be substituted by halogen, lower alkyl or lower alkoxy; and

more preferably R is a lower alkyl group, a phenyl group that may optionally be substituted by methyl or methoxy, a cyclohexyl group that may optionally be substituted by chlorine, or a benzyl group that may optionally be substituted by methyl or methoxy.

Typical examples of the compound of formula (I) provided by this invention are as follows, although the scope of this invention is not limited thereto.

(1) 4-hydroxy-3-methyl-3'-methyl-4'-methoxydiphenylsulfone
(2) 4-hydroxy-3-methyl-4'-methoxydiphenylsulfone
(3) 4-hydroxy-3'-methyl-4'-methoxydiphenylsulfone
(4) 4-hydroxy-2-methyl-2'-methyl-4'-methoxydiphenylsulfone
(5) 4-hydroxy-2-methyl-4'-methoxydiphenylsulfone
(6) 4-hydroxy-2'-methyl-4'-methoxydiphenylsulfone
(7) 4-hydroxy-3-methyl-2'-methyl-4'-methoxydiphenylsulfone
(8) 4-hydroxy-2-methyl-3'-methyl-4'-methoxydiphenylsulfone
(9) 4-hydroxy-3-methyl-3'-methyl-4'-methoxydiphenylsulfone
(10) 4-hydroxy-3-methyl-4'-ethoxydiphenylsulfone
(11) 4-hydroxy-3'-methyl-4'-ethoxydiphenylsulfone
(12) 4-hydroxy-2-methyl-2'-methyl-4'-ethoxydiphenylsulfone
(13) 4-hydroxy-2-methyl-4'-ethoxydiphenylsulfone
(14) 4-hydroxy-2'-methyl-4'-ethoxydiphenylsulfone
(15) 4-hydroxy-3-methyl-2'-methyl-4'-ethoxydiphenylsulfone
(16) 4-hydroxy-2-methyl-3'-methyl-4'-ethoxydiphenylsulfone
(17) 4-hydroxy-3-methyl-3'-methyl-4'-propoxydiphenylsulfone
(18) 4-hydroxy-3-methyl-4'-propoxydiphenylsulfone
(19) 4-hydroxy-3'-methyl-4'-propoxydiphenylsulfone
(20) 4-hydroxy-2-methyl-2'-methyl-4'-propoxydiphenylsulfone
(21) 4-hydroxy-2-methyl-4'-propoxydiphenylsulfone

(22) 4-hydroxy-2'-methyl-4'-propoxydiphenylsulfone
(23) 4-hydroxy-3-methyl-2'-methyl-4'-propoxydiphenylsulfone
(24) 4-hydroxy-2-methyl-3'-methyl-4'-propoxydiphenylsulfone
(25) 4-hydroxy-3-methyl-3'-methyl-4'-propoxydiphenylsulfone
(26) 4-hydroxy-3-methyl-4'-benzyloxydiphenylsulfone
(27) 4-hydroxy-3'-methyl-4'-benzyloxydiphenylsulfone
(28) 4-hydroxy-2-methyl-2'-methyl-4'-benzyloxydiphenylsulfone
(29) 4-hydroxy-2-methyl-4'-benzyloxydiphenylsulfone
(30) 4-hydroxy-2'-methyl-4'-benzyloxydiphenylsulfone
(31) 4-hydroxy-3-methyl-2'-methyl-4'-benzyloxydiphenylsulfone
(32) 4-hydroxy-2-methyl-3'-methyl-4'-benzyloxydiphenylsulfone
(33) 4-hydroxy-3-phenyl-4'-vinyloxydiphenylsulfone
(34) 4-hydroxy-2'-phenyl-4'-phenoxydiphenylsulfone
(35) 4-hydroxy-3-phenyl-2'-phenyl-4'-p-methylphenoxydiphenylsulfone
(36) 4-hydroxy-3-phenyl-3'-phenyl-4'-cyclohexyloxydiphenylsulfone
(37) 4-hydroxy-3-phenyl-4'-(4-methylcyclohexyloxy)diphenylsulfone
(38) 4-hydroxy-2,5-diphenyl-4'-benzyloxydiphenylsulfone
(39) 4-hydroxy-2',5'-diphenyl-4'-p-methylbenzyloxydiphenylsulfone
(40) 4-hydroxy-2,5-diphenyl-2'-phenyl-4'-butoxydiphenylsulfone
(41) 4-hydroxy-3-p-tolyl-4'-vinyloxydiphenylsulfone
(42) 4-hydroxy-2'-p-methoxyphenyl-4'-phenoxydiphenylsulfone
(43) 4-hydroxy-3-p-chlorophenyl-2'-phenyl-4'-p-methoxyphenoxydiphenylsulfone
(44) 4-hydroxy-3-p-nitrophenyl-3'-phenyl-4'-cyclohexyloxydiphenylsulfone
(45) 4-hydroxy-3-p-aminophenyl-4'-(4-methoxycyclohexyloxy)diphenylsulfone
(46) 4-hydroxy-2-m-nitrophenyl-5-o-chlorophenyl-4'-benzyloxydiphenylsulfone
(47) 4-hydroxy-2'-o-chlorophenyl-5'-p-methoxyphenyl-4'-p-methoxybenzyloxydiphenylsulfone
(48) 4-hydroxy-2,5-di-p-tolyl-2'-p-tolyl-4'-butoxydiphenylsulfone
(49) 4-hydroxy-3-cyclohexyl-4'-vinyloxydiphenylsulfone
(50) 4-hydroxy-2'-cyclohexyl-4'-phenoxydiphenylsulfone
(51) 4-hydroxy-3-cyclohexyl-2'-cyclohexyl-4'-p-chlorophenoxydiphenylsulfone
(52) 4-hydroxy-3-cyclohexyl-3'-cyclohexyl-4'-cyclohexyloxydiphenylsulfone
(53) 4-hydroxy-3-cyclohexyl-4'-(4-chlorocyclohexyloxy)diphenylsulfone
(54) 4-hydroxy-2,5-dicyclohexyl-4'-benzyloxydiphenylsulfone
(55) 4-hydroxy-2',5'-dicyclohexyl-4'-p-chlorobenzyloxydiphenylsulfone
(56) 4-hydroxy-2,5-dicyclohexyl-2'-cyclohexyl-4'-butoxydiphenylsulfone
(57) 4-hydroxy-3-cyclopentyl-4'-vinyloxydiphenylsulfone
(58) 4-hydroxy-2'-cyclopentyl-4'-phenoxydiphenylsulfone
(59) 4-hydroxy-3-cyclopentyl-2'-cyclopentyl-4'-p-nitrophenoxydiphenylsulfone
(60) 4-hydroxy-3-cyclopentyl-3'-cyclopentyl-4'-cyclohexyloxydiphenylsulfone
(61) 4-hydroxy-3-cyclopentyl-4'-(4-nitrocyclohexyloxy)diphenylsulfone
(62) 4-hydroxy-2,5-dicyclopentyl-4'-benzyloxydiphenylsulfone
(63) 4-hydroxy-2',5'-dicyclopentyl-4'-p-nitrobenzyloxydiphenylsulfone
(64) 4-hydroxy-2,5-dicyclopentyl-2'-cyclopentyl-4'-butoxydiphenylsulfone
(65) 4-hydroxy-3-ethyl-4'-vinyloxydiphenylsulfone
(66) 4-hydroxy-2'-ethyl-4'-phenoxydiphenylsulfone
(67) 4-hydroxy-3-ethyl-2'-ethyl-4'-p-methylphenoxydiphenylsulfone
(68) 4-hydroxy-3-ethyl-3'-ethyl-4'-cyclohexyloxydiphenylsulfone
(69) 4-hydroxy-3-ethyl-4'-(4-methylcyclohexyloxy)diphenylsulfone
(70) 4-hydroxy-2,5-diethyl-4'-benzyloxydiphenylsulfone
(71) 4-hydroxy-2',5'-diethyl-4'-p-methylbenzyloxydiphenylsulfone
(72) 4-hydroxy-2,5-diethyl-2'-ethyl-4'-butoxydiphenylsulfone
(73) 4-hydroxy-3-propyl-4'-vinyloxydiphenylsulfone
(74) 4-hydroxy-2'-propyl-4'-phenoxydiphenylsulfone
(75) 4-hydroxy-3-propyl-2'-propyl-4'-p-methoxyphenoxydiphenylsulfone
(76) 4-hydroxy-3-propyl-3'-propyl-4'-cyclohexyloxydiphenylsulfone
(77) 4-hydroxy-3-propyl-4'-(4-methoxycyclohexyloxy)diphenylsulfone
(78) 4-hydroxy-2,5-dipropyl-4'-benzyloxydiphenylsulfone
(79) 4-hydroxy-2',5'-dipropyl-4'-(4-methoxybenzyloxy)diphenylsulfone

(80) 4-hydroxy-2,5-dipropyl-2'-propyl-4'-butoxydiphenylsulfone
(81) 4-hydroxy-3-ethyl-4'-methoxydiphenylsulfone
(82) 4-hydroxy-2'-ethyl-4'-methoxydiphenylsulfone
(83) 4-hydroxy-3-ethyl-2'-ethyl-4'-methoxydiphenylsulfone
(84) 4-hydroxy-3-ethyl-3'-ethyl-4'-methoxydiphenylsulfone
(85) 4-hydroxy-3-ethyl-4'-methoxydiphenylsulfone
(86) 4-hydroxy-2,5-diethyl-4'-ethoxydiphenylsulfone
(87) 4-hydroxy-2',5'-diethyl-4'-ethoxydiphenylsulfone
(88) 4-hydroxy-2,5-diethyl-2'-ethyl-4'-ethoxydiphenylsulfone
(89) 4-hydroxy-3-propyl-4'-propoxydiphenylsulfone
(90) 4-hydroxy-2'-propyl-4'-propoxydiphenylsulfone
(91) 4-hydroxy-3-propyl-2'-propyl-4'-propoxydiphenylsulfone
(92) 4-hydroxy-3-propyl-3'-propyl-4'-propoxydiphenylsulfone
(93) 4-hydroxy-3-propyl-4'-butoxydiphenylsulfone
(94) 4-hydroxy-2,5-dipropyl-4'-butoxydiphenylsulfone
(95) 4-hydroxy-2',5'-dipropyl-4'-butoxydiphenylsulfone
(96) 4-hydroxy-2,5-dipropyl-2'-propyl-4'-butoxydiphenylsulfone
(97) 4-hydroxy-3-methyl-4'-propoxydiphenylsulfone
(98) 4-hydroxy-3-methyl-3'-methyl-4'-propoxydiphenylsulfone
(99) 4-hydroxy-3-methyl-3'-methyl-4'-pentyloxydiphenylsulfone
(100) 4-hydroxy-3-methyl-3'-methyl-4'-cyclohexyloxydiphenylsulfone
(101) 4-hydroxy-3-methyl-3'-methyl-4'-benzyloxydiphenylsulfone
(102) 4-hydroxy-3-methyl-3'-methyl-4'-m-methylbenzyloxydiphenylsulfone
(103) 4-hydroxy-3-methyl-3'-methyl-4'-butoxydiphenylsulfone
(104) 4-hydroxy-3-methyl-3'-methyl-4'-isopropoxydiphenylsulfone
(105) 4-hydroxy-3-methyl-3'-methyl-4'-isobutoxydiphenylsulfone
(106) 4-hydroxy-3-ethyl-4'-ethoxydiphenylsulfone
(107) 4-hydroxy-3-ethyl-4'-propoxydiphenylsulfone
(108) 4-hydroxy-3-isopropyl-4'-ethoxydiphenylsulfone
(109) 4-hydroxy-3-isopropyl-4'-propoxydiphenylsulfone
(110) 4-hydroxy-3-cyclohexyl-4'-ethoxydiphenylsulfone

The compound of formula (I) in this invention can be produced by, according to, e.g., the following reaction scheme A,

Reaction Scheme (A)

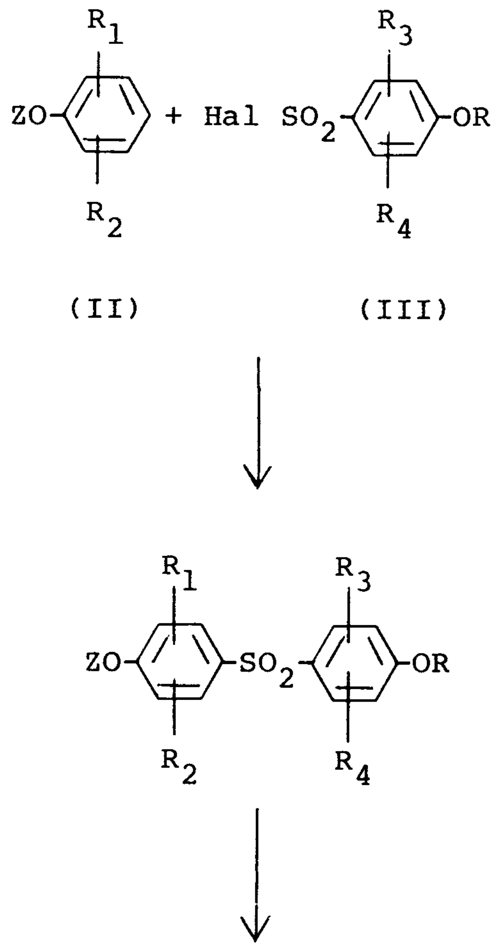

(II) (III)

(IV)

Compound of formula (I)

wherein

Z denotes a protective group of a hydroxyl group, for example, an acyl group such as acetyl,

Hal denotes a halogen atom such as chlorine, and

$R_1$, $R_2$, $R_3$ and $R_4$ are as defined above, reacting a phenol derivative of formula (II) with a p-alkoxybenzenesulfonyl halide derivative of formula (III), and removing the protective group of the hydroxyl group from the resultant compound of formula (IV).

The reaction of the compound of formula (II) with the compound of formula (III) can be usually carried out in an inert solvent, optionally in the presence of a Lewis acid catalyst at a temperature of about -20 °C to 80 °C, preferably -10 °C to 30 °C. Examples of the inert solvent include saturated aliphatic hydrocarbons such as hexane, cyclohexane and heptane; halogenated aliphatic hydrocarbons such as dichloromethane, chloroform, carbon tetrachloride, dichloroethane and trichloroethane; aromatic hydrocarbons such as chlorobenzene, o-dichlorobenzene, 1,2,4-trichlorobenzene and nitrobenzene; and aliphatic nitro compounds such as nitromethane and nitroethane.

Examples of the Lewis acid catalyst that can be used here include aluminum chloride, zinc chloride, stannic chloride, ferric chloride, titanium chloride and boron trifluoride. The amount of the Lewis acid catalyst is usually 5 to 100 % by weight, preferably 10 to 30 % by weight based on the compound of formula (III).

The proportion of the compound of formula (III) based on the compound of formula (II) is not strictly limited. It is however convenient that the proportion of the compound of formula (III) is usually 1.0 to 1.3 mols, preferably 1.0 to 1.1 mols per mol of the compound of formula (II).

The compound of formula (I) can be obtained by subjecting the compound of formula (IV) formed via the above reaction to the removal reaction of the hydroxyl protective group known per se, for example, the alkaline hydrolysis.

Alternatively, the compound of formula (I) can be produced by, for example, according to the following

reaction scheme B,

Reaction Scheme (B)

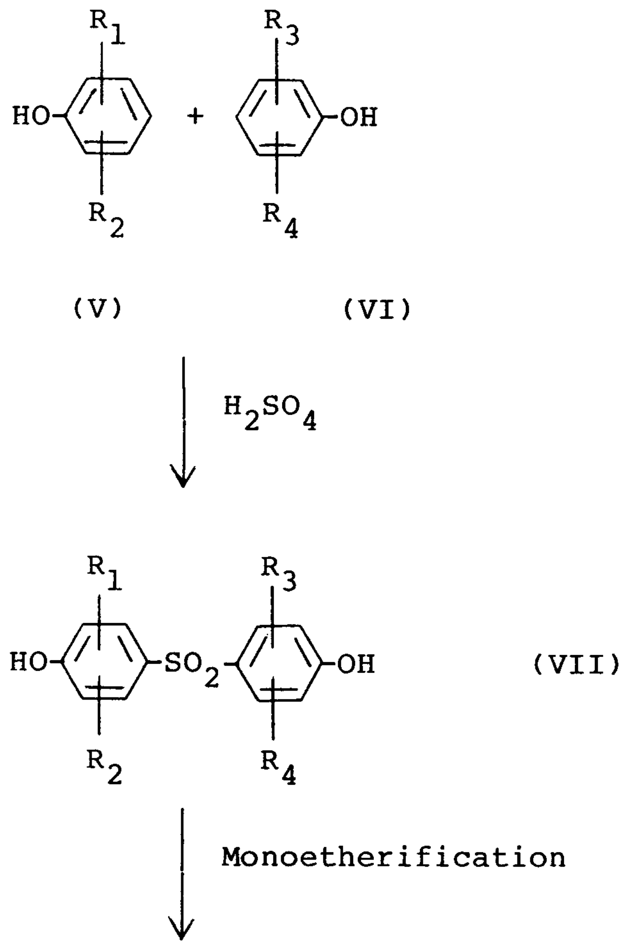


Compound of formula (I)

wherein,

$R_1$, $R_2$, $R_3$ and $R_4$ are as defined above, reacting the phenol derivatives of formulas (V) and (VI) with sulfonic acid to form the 4,4'-dihydroxydiphenylsulfone derivative of formula (VII) and then monoetherifying it.

The sulfonation with sulfuric acid of the phenol derivatives of formulas (V) and (VI) at the first stage of the reaction shown by the above reaction scheme B can be carried out by a dehydration reaction known per se [see J. Chem. Soc., 43 (1934), U.S. Patent 2,122,958, Monatsh. Chem., 91, 57 (1960), U.S. Patent 3,383,421, and J. Prakt. Chem., 137, 216 (1933].

The thus obtained 4,4'-dihydroxydiphenylsulfone derivative of formula (VII) can be formed into the compound of formula (I) at the second stage by monoetherification with the use of, e.g., a compound of formula (VIII)

RX (VIII)

wherein

X denotes a halogen atom, preferably a bromine atom, and

R is as defined above.

The reaction of the compound of formula (VII) with the compound of formula (VIII) can usually be effected in a solvent inert to the reaction, preferably in the presence of a base at a temperature of 40 to

200 ° C, preferably 60 to 150 ° C. Examples of the inert solvent include N,N-dimethylformamide, dimethyl sulfoxide, pyridine, acetone, methyl isobutyl ketone, acetophenone and water.

Examples of the base that can be used here include sodium hydroxide, potassium hydroxide, potassium carbonate, sodium hydrogencarbonate, sodium methylate and sodium hydride. The amount of the base is 0.5 to 1.3 mols, preferably 0.8 to 1.1 mols per mol of the compound of formula (VII).

The amount of the compound of formula (VIII) is not strictly limited, but usually 0.5 to 1.3 mols, preferably 0.8 to 1.1 mols per mol of the compound of formula (VII).

The thus obtained compound of formula (I) can be isolated and purified by a method known per se, such as chromatography, recrystallization or extraction with a solvent.

The 4-hydroxydiphenylsulfone derivative of formula (I) provided by this invention has a characteristic that for example, solubility in oil ink is low, and it is useful as a developer in a heat sensitive color formation layer of a heat sensitive recording material.

In another aspect of this invention, there is provided a heat sensitive recording material having a heat sensitive color formation layer comprising as main components a colorless or light-colored basic leuco dye and an organic developer on a support, characterized in that the organic developer comprises at least one compound of formula (I).

The heat sensitive recording material will be described in more detail below.

Heat sensitive color formation layer

The basic leuco dye used in the heat sensitive recording material of this invention is not limited in particular, and any conventional heat sensitive recording materials are available. For example, triphenylmethane-type, fluoran-type and fluorene-type leuco dyes are preferable. Concrete examples thereof are as follows.

Triphenylmethane-type leuco dyes

3,3-bis(p-diemthylaminophenyl)-6-dimethylaminophthalide [Crystal Violet Lactone]

Fluoran-type leuco dyes

3-dimethylamino-6-methyl-7-anilinofluoran
3-(N-ethyl-p-toluidino)-6-methyl-7-anilinofluoran
3-(N-ethyl-N-isoamylamino)-6-methyl-7-anilinofluoran
3-diethylamino-6-methyl-7-(o,p-dimethylanilino)fluoran
3-pyrrolidino-6-methyl-7-anilinofluoran
3-piperidino-6-methyl-7-anilinofluoran
3-(N-cyclohexyl-N-methylamino)-6-methyl-7-anilinofluoran
3-diethylamino-7-(m-trifluoromethylanilino)-fluoran
3-N-n-dibutylamino-6-methyl-7-anilinofluoran
3-N-n-dibutylamino-7-(o-chloroanilino)fluoran
3-(N-ethyl-N-tetrahydrofurfurylamino)-6-methyl-7-anilinofluoran
3-dibutylamino-6-methyl-7-(o,p-dimethylanilino)fluoran
3-(N-methyl-N-propylamino)-6-methyl-7-anilinofluoran
3-dibutylamino-7-(o-chloroanilino)fluoran
3-diethylamino-7-(o-chloroanilino)fluoran
3-diethylamino-6-methylchlorofluoran
3-diethylamino-6-methylfluoran
3-cyclohexylamino-6-chlorofluoran
3-diethylaminobenzo-[a]-fluoran
3-n-dipentylamino-6-methyl-7-anilinofluoran
2-(4-oxahexyl)-3-dimethylamino-6-methyl-7-anilinofluoran
2-(4-oxahexyl)-3-diethylamino-6-methyl-7-anilinofluoran
2-(4-oxahexyl)-3-dipropylamino-6-methyl-7-anilinofluoran

Fluorene-type leuco dyes 3,6,6'-tris(dimethylamino)spiro[fluorene-9,3'-phthalide]
3,6,6'-tris(diethylamino)spiro[fluorene-9,3'-phthalide]

These dyes may be used either singly or in combination.

The heat sensitive color formation layer of the heat sensitive recording material in this invention can contain a sensitizer, thereby making it possible to more improve resistance to oil ink. Examples of the sensitizer include 1,2-di(3-methylphenoxy)ethane, di-(p-methylbenzyl) oxalate, p-benzylbiphenyl, betabenzyloxynaphthalene, 4-biphenyl-p-tolyl ether, m-terphenyl, 1,2-diphenoxyethane, dibenzyl oxalate and di-(p-chlorobenzyl) oxalate.

Said heat sensitive color formation layer may further contain an image stabilizer. Examples of the image stabilizer are as follows.

4,4'-butylidene(6-tert-butyl-3-methylphenol)
2,2'-di-tert-butyl-5,5'-dimethyl-4,4'-sulfonyldiphenol
1,1,3-tris(2-methyl-4-hydroxy-5-cyclohexylphenyl)butane
1,1,3-tris(2-methyl-4-hydroxy-5-tert-butylphenyl)
butane

Moreover, the heat sensitive color formation layer according to this invention can contain, as required, an organic or inorganic filler such as silica, calcium carbonate, kaolin, calcined kaolin, diatomaceous earth, talc, titanium oxide, or aluminum hydroxide; a mold release agents such as an aliphatic acid metal salt; a lubricant such as a wax; a benzophenone-type or triazole-type UV absorber; a water resistance imparting agent such as glyoxal; a dispersant; and a defoamer.

A binder used to apply the aforesaid heat sensitive color formation component to the support can be any binder ordinarily used in the heat sensitive recording material. Examples of the binder are modified polyvinyl alcohols having a polymerization degree of 200 to 1900, such as a completely saponified polyvinyl alcohol, a partially saponified polyvinyl alcohol, a carboxy-modified polyvinyl alcohol, an amide-modified polyvinyl alcohol, a sulfonic acid-modified polyvinyl alcohol, and a butyral-modified polyvinyl alcohol; cellulose derivatives such as hydroxyethyl cellulose, methyl cellulose, carboxymethyl cellulose, ethyl cellulose, and acetyl cellulose; copolymers such as a styrene-maleic anhydride copolymer, and a styrenebutadiene copolymer; homopolymers or copolymers of monomers such as vinyl chloride, vinyl acetate, acrylamide, acrylic acid ester, vinyl butyral, and styrene; and resins such as a polyamide resin, a silicone resin, a petroleum resin, a terpene resin, a ketone resin and a coumarone resin. These high-molecular materials can be used by being dissolved in solvents such as water, alcohols, ketones, esters and hydrocarbons, or by being emulsified or dispersed in a paste form in water or other media.

In the heat sensitive recording sheet of this invention, the amounts of the organic developer and the basic leuco dye and the types and the amounts of the other components are determined according to required performance and recording adaptability, and are not limited in particular. The amount of the organic developer is usually 1 to 8 parts by weight, preferably 1.5 to 3 parts by weight per part by weight of the basic leuco dye. The amount of the sensitizer is usually 0.1 to 5 parts by weight, preferably 0.3 to 3 parts by weight per part by weight of the basic leuco dye. The amount of the image stabilizer is usually 0.1 to 3 parts by weight, preferably 0.2 to 2.5 parts by weight per part by weight of the basic leuco dye. The amount of the filler is usually 1 to 20 parts by weight, preferably 5 to 10 parts by weight per part by weight of the basic leuco dye. The amount of the binder is usually 10 to 25 % by weight, preferably 10 to 20 % by weight based on the total solids content.

Support

The support that can be used in the heat sensitive recording material of this invention is not limited in particular. Examples thereof are paper, synthetic paper and a synthetic film.

Heat sensitive recording material

The heat sensitive recording material can be obtained by applying to the support a coating solution formed from the basic leuco dye, the compound of formula (I) as an organic developer, the sensitizer and the binder. The organic developer, the basic leuco dye and optionally the additive components can be pulverized to a particle diameter of less than several microns by means of a pulverizer such as a ball mill, an attritor or a sand grinder, or a suitable emulsifier, and adding a binder and various additives according to purposes to form a coating solution.

The thickness of the formed heat sensitive color formation layer is not particularly limited and can vary over a wide range depending to usage, etc.; it is usually about 3 to 9 microns.

Further, an undercoat layer of a high-molecular material containing a filler can be formed under the heat sensitive color formation layer to increase color sensitivity.

Still further, an overcoat layer of a high-molecular material can be formed on the heat sensitive color

formation layer to increase preservability.

The heat sensitive recording material of this invention using the novel phenolic compound of this invention as the developer does not cause discoloration even by writing with an oil ink. The reasons are as follows.

Generally, when a heat sensitive recording material is written with a felt pen of an oil ink, a developer, a leuco dye and a sensitizer of a heat sensitive color formation layer are dissolved in the organic solvent contained in the ink. As the solvent is evaporated, the color formation composition containing the developer, the leuco dye and the sensitizer is formed on the written portion. When using a red ink, for example, the red color and the formed color of the heat sensitive recording material are mixed to give a dull written image.

Meanwhile, in the heat sensitive recording material of this invention, the 4-hydroxydiphenylsulfone derivative containing specific substituents in the two benzene rings adjacent the sulfone group is used as the developer, or the specific sensitizer is further used conjointly. In consequence, it is thought that since solubility of these materials in the solvent is extremely low and a color formation composition is little formed even by evaporation of the solvent, discoloration owing to the oil ink does not occur.

Accordingly, the heat sensitive recording material using the phenolic compound of this invention as the developer can freely be written with the oil ink. Besides, said material is excellent in heat response, and color recording in a high concentration can be provided.

In the heat sensitive recording material of this invention, a speed at which to melt, dissolve or diffuse the developer in the sensitizer and saturation solubility are extremely high; a speed at which to melt, dissolve or diffuse the dye in the sensitizer and saturation solubility are also high. Therefore, when the organic developer is reacted with the sensitizer and the leuco dye by heating physically and chemically, the color formation composition is rapidly formed, and a color formation image in a high concentration is obtained.

The following Examples illustrate this invention more specifically. In said Examples, parts are all by weight.

SYNTHESIS EXAMPLE 1

Synthesis of 4-hydroxy-3-methyl-4'-ethoxydiphenylsulfone

This compound was prepared by the following three-stage synthesis method using ethoxybenzene as a starting material.

(1) Synthesis of p-ethoxybenzenesulfonyl chloride

One hundred grams of ethoxybenzene was added to 600 ml of chloroform, and 191 g of chlorosulfonic acid was added dropwise at 5°C or below under ice cooling. After the dropwise addition, the temperature was returned to room temperature, and stirring was conducted for 1 hour to terminate the reaction. The reaction solution was charged into ice water and the organic layer was separated. Then, the solvent was evaporated, and the residue was purified by a silica gel column (eluent: n-hexane/ethyl acetate = 10/1) to obtain 110.9 g of p-ethoxybenzenesulfonyl chloride.

(2) Synthesis of 3-methyl-4-acetoxy-4'-ethoxydiphehylsulfone

o-Tolyl acetate (68.3 g) was added to 300 ml of 1,2-dichloroethane, and 18.0 g of ferric chloride was then charged therein. To the solution was added dropwise 110.3 g of the product in (1) which was dissolved in 200 ml of 1,2-dichloroethane. After the dropwise addition, stirring was conducted for 4 days to terminate the reaction. The reaction solution was charged in water and extracted with chloroform. The extract was washed with water, and the solvent was then evaporated. The residue was purified with a silica gel column (eluent: n-hexane/ethyl acetate = 3/1) to obtain 7.1 g of 3-methyl-4-acetoxy-4'-ethoxydiphenylsulfone.

(3) Synthesis of 4-hydroxy-3-methyl-4'-ethoxydiphenylsulfone

The product (7.1 g) in (2) was dissolved in 70 ml of ethanol, and 0.9 g of sodium hydroxide dissolved in 10 ml of water was added. Stirring was conducted for 3 hours to terminate the reaction. The reaction solution was charged in water and extracted with ethyl acetate. The extract was then washed with water and the solvent was evaporated. The residue was purified by a silica gel column (eluent: n-hexane/ethyl acetate = 7/3) to obtain 2.4 g of final 4-hydroxy-3-methyl-4'-ethoxydiphenylsulfone (white crystal, m.p. 158 to

159° C).

SYNTHESIS EXAMPLE 2

Synthesis of 4-hydroxy-3'-methyl-4'-ethoxydiphenylsulfone

This compound was formed by the following 3-stage synthesis method using 2-ethoxytoluene as a starting material.

(1) Synthesis of 3-methyl-4-ethoxybenzenesulfonyl chloride

To 230 ml of chloroform was added 23.3 g of 2-ethoxytoluene, and 39.9 g of chlorosulfonic acid was added dropwise at 0° C or lower under ice cooling. After the dropwise addition, the temperature was returned to room temperature. Stirring was conducted overnight to terminate the reaction. The reaction solution was charged in ice water to separate the organic layer, followed by evaporating the solvent. There was obtained 31.5 g of 2-methyl-4-ethoxybenzenesulfonyl chloride.

(2) Synthesis of 4-tosyloxy-3'-methyl-4'-ethoxydiphenylsulfone

To 50 ml of 1,2-dichloroethane was added 5.8 g of phenol tosylate, and 4.3 g of ferric chloride was then charged. Five grams of the product in (1) which was dissolved in 50 ml of 1,2-dichloroethane was added dropwise at 10° C. After the dropwise addition, the mixture was stirred overnight to terminate the reaction. The reaction solution was charged in water and extracted with chloroform. After the extract was washed with water, the solvent was evaporated and the residue was purified by a silica gel column (eluent: n-hexane/ethyl acetate = 3/1) to obtain 1.1 g of 4-tosyloxy-3'-methyl-4'-ethoxydiphenylsulfone.

(3) Synthesis of 4-hydroxy-3'-methyl-4'-ethoxydiphenylsulfone

The product (1.1 g) in (2) was dissolved in 10 ml of ethanol, and 0.17 g of potassium hydroxide dissolved in 5 ml of water was added thereto. The solution was stirred at 50° C for 2 hours to terminate the reaction. The reaction solution was charged in water, and extracted with ethyl acetate. The extract was then washed with water, and the solvent was evaporated. The residue (0.7 g) was crystallized with n-hexane to obtain 0.1 g of final 4-hydroxy-3'-methyl-4'-ethoxydiphenylsulfone (white crystal, m.p. 158 to 159° C).

SYNTHESIS EXAMPLE 3

Synthesis of 4-hydroxy-3-methyl-3'-methyl-4'-propoxydiphenylsulfone

This compound was formed by the following 3-stage synthesis method using 2-propoxytoluene as a starting material.

(1) Synthesis of 3-methyl-4-propoxybenzenesulfonyl chloride

3-Methyl-4-propoxybenzenesulfonyl chloride was formed as in Synthesis Example 1 except that ethoxybenzene in (1) of Synthesis Example 1 was replaced with 2-propoxytoluene.

(2) Synthesis of 3-methyl-4-acetoxy-3'-methyl-4'-propoxydiphenylsulfone

3-Methyl-4-acetoxy-3'-methyl-4'-propoxydiphenylsulfone was formed as in Synthesis Example 1 except that p-ethoxybenzenesulfonyl chloride in (2) of Synthesis Example 1 was replaced with 3-methyl-4-propoxybenzenesulfonyl chloride.

(3) Synthesis of 4-hydroxy-3-methyl-3'-methyl-4'-propoxydiphenylsulfone

4-Hydroxy-3-methyl-3'-methyl-4'-propoxydiphenylsulfone (white crystal, m.p. 137° C) was prepared as in Synthesis Example 1 except that 3-methyl-4-acetoxy-4'-ethoxydiphenylsulfone in (2) of Synthesis Example 1 was replaced with 3-methyl-4-acetoxy-3'-methyl-4'-propoxydiphenylsulfone.

SYNTHESIS EXAMPLE 4

Synthesis of 4-hydroxy-3-methyl-3'-methyl-4-methoxydiphenylsulfone

This compound was formed by the following three-stage synthesis method using 2-methoxytoluene as a starting material.

(1) Synthesis of 3-methyl-4-methoxybenzenesulfonyl chloride

3-Methyl-4-methoxybenzenesulfonyl chloride was formed as in Synthesis Example 1 except that ethoxybenzene in (1) of Synthesis Example 1 was replaced with 2-methoxytoluene.

(2) Synthesis of 3-methyl-4-acetoxy-3'-methyl-4'-methoxydiphenylsulfone

3-Methyl-4-acetoxy-3'-methyl-4'-methoxydiphenylsulfone was formed as in Synthesis Example 1 except that p-ethoxybenzenesulfonyl chloride in (2) of Synthesis Example 1 was replaced with 3-methyl-4-methoxybenzenesulfonyl chloride.

(3) Synthesis of 4-hydroxy-3-methyl-3'-methyl-4'-methoxydiphenylsulfone

4-Hydroxy-3-methyl-3'-methyl-4'-methoxydiphenylsulfone (white crystal, m.p. 184 to 185 °C) was formed as in Synthesis Example 1 except that 3-methyl-4-acetoxy-4'-ethoxydiphenylsulfone in (3) of Synthesis Example 1 was replaced with 3-methyl-4-acetoxy-3'-methyl-4'-methoxydiphenylsulfone.

SYNTHESIS EXAMPLE 5

Synthesis of 4-hydroxy-3-methyl-4'-n-propoxydiphenylsulfone

Using 111.5 g of propoxybenzene as a starting material, 3.7 g of 4-hydroxy-3-methyl-4'-n-propoxydiphenylsulfone (m.p. 136.5 to 139.0 °C) was obtained by the 3-stage reaction as in Synthesis Example 1.

SYNTHESIS EXAMPLE 6

The compound in Synthesis Example 6 was formed by forming 3,3'-dimethyl-4,4'-dihydroxydiphenylsulfone from o-cresol and then monoetherifying it, according to a known method [a method described in J. Chem. Soc., 43 (1934) or U.S. Patent 2,122,958 (1936)].

Synthesis of 4-hydroxy-3-methyl-3'-methyl-4'-isopropoxydiphenylsulfone

3,3'-Dimethyl-4,4'-dihydroxydiphenylsulfone (27.8 g) and 4.0 g of sodium hydroxide were dissolved in DMSO, and 12.3 g of isopropyl bromide was then added. The mixture was reacted at room temperature for 6 hours. After the reaction was over, toluene and water were added to the solution, and they were stirred upon heating. After the aqueous layer was separated, the toluene layer was washed with water several times and then with a sodium hydrogencarbonate solution. When the toluene layer was neutralized with dilute hydrochloric acid and cooled, crystals were precipitated. The crystals were separated by filtration and recrystallized with toluene to obtain 23.5 g of crystals (m.p. 183.0 to 184.0 °C)

SYNTHESIS EXAMPLE 7

Synthesis of 4-hydroxy-3-methyl-3'-methyl-4'-butoxydiphenylsulfone

Synthesis Example 6 was repeated except that 12.3 g of isopropyl bromide was replaced with 13.7 g of butyl bromide. There was obtained 25.4 g of 4-hydroxy-3-methyl-3'-methyl-4'-butoxydiphenylsulfone (m.p. 152.5 to 155.0 °C).

SYNTHESIS EXAMPLE 8

Synthesis of 4-hydroxy-3-methyl-3'-methyl-4'-pentyloxydiphenylsulfone

Synthesis Example 6 was repeated except that 12.3 g of isopropyl bromide was replaced with 15.1 g of pentyl bromide. There was obtained 25.1 g of 4-hydroxy-4-methyl-3'-methyl-4'-pentyloxydiphenylsulfone (m.p. 95.5 to 100.0 °C).

SYNTHESIS EXAMPLE 9

Synthesis of 4-hydroxy-3-methyl-3'-methyl-4'-cyclohexyloxydiphenylsulfone

Synthesis Example 6 was repeated except that 12.3 g of isopropyl bromide was replaced with 16.3 g of cyclohexyl bromide. There was obtained 13.3 g of 4-hydroxy-4-methyl-3'-methyl-4'-cyclohexyloxydiphenylsulfone (m.p. 143.0 to 149.0 °C).

SYNTHESIS EXAMPLE 10

Synthesis of 4-hydroxy-3-methyl-3'-methyl-4'-benzyloxydiphenylsulfone

Synthesis Example 6 was repeated except that 12.3 g of isopropyl bromide was replaced with 12.6 g of benzyl chloride. There was obtained 29.1 g of 4-hydroxy-3-methyl-3'-methyl-4'-benzyloxydiphenylsulfone (m.p. 145.5 to 147.0 °C).

SYNTHESIS EXAMPLE 11

Synthesis of 4-hydroxy-3-methyl-3'-methyl-4'-m-methylbenzyloxydiphenylsulfone

Synthesis Example 6 was repeated except that 12.3 g of isopropyl bromide was replaced with 14.1 g of m-methylbenzyl chloride. There was obtained 29.7 g of 4-hydroxy-3-methyl-3'-methyl-4'-m-methylbenzyloxydiphenylsulfone (m.p. 141.5 to 144.0 °C).

The other compounds of this invention which were prepared as above are shown in Table 1.

Table 1

| Synthesis Example No. | Phenolic compound | m.p. (°C) |
|---|---|---|
| 12 | 4-hydroxy-3-methyl-4'-methoxydiphenyl-sulfone | 162-163 |
| 13 | 4-hydroxy-3-methyl-3'-methyl-4'-ethoxy-diphenylsulfone | 189 |
| 14 | 4-hydroxy-3-methyl-3'-methyl-4'-iso-butoxydiphenylsulfone | 105.0-112.5 |
| 15 | 4-hydroxy-2-methyl-2'-methyl-4'-methoxy-diphenylsulfone | 140-142 |
| 16 | 4-hydroxy-2-methyl-2'-methyl-4'-ethoxy-diphenylsulfone | 148-151 |
| 17 | 4-hydroxy-3-ethyl-4'-ethoxydiphenyl-sulfone | 139-140 |
| 18 | 4-hydroxy-3-ethyl-4'-propoxydiphenyl-sulfone | 122-124 |
| 19 | 4-hydroxy-3-isopropyl-4'-ethoxydi-phenylsulfone | 138-139 |
| 20 | 4-hydroxy-3-isopropyl-4'-propoxydi-phenylsulfone | 131-133 |
| 21 | 4-hydroxy-3-cyclohexyl-4'-ethoxydi-phenylsulfone | 176-178 |

Examples of producing heat sensitive recording sheets using the phenolic compounds of this invention will be described below.

EXAMPLES 1 to 6

| Solution A (developer dispersion) | parts |
|---|---|
| developer (see Table 2) | 6.0 |
| 10 % polyvinyl alcohol aqueous solution | 18.8 |
| water | 11.2 |
| Solution B (dye dispersion) | |
| 3-N-n-dibutylamino-6-methyl-7-anilino-fluoran | 2.0 |
| 10 % polyvinyl alcohol aqueous solution | 4.6 |
| water | 2.6 |

Each of the solutions having the above formulations was pulverized to an average particle diameter of 1 micron by a sand grinder. Then the dispersions were mixed in the followingproportions to form a coating solution.

| | parts |
|---|---|
| solution A | 36.0 |
| solution B | 9.2 |
| kaolin clay (50 % dispersion) | 12.0 |

The above coating solution was coated on one side of a base sheet (50 g/m$^2$) such that the coating amount reached 6.0 g/m$^2$, and dried. The resultant sheet was treated with a supercalender such that smoothness became 500 to 600 seconds.

COMPARATIVE EXAMPLES 1 to 4

| Solution E (developer dispersion) | parts |
|---|---|
| developer (see Table 2) | 6.0 |
| 10 % polyvinyl alcohol aqueous solution | 18.8 |
| water | 11.2 |
| Solution B (dye dispersion) | parts |
| 3-N-n-dibutylamino-6-methyl-7-anilino-fluoran | 2.0 |
| 10 % polyvinyl alcohol aqueous solution | 4.6 |
| water | 2.6 |

Each of the solutions having the above formulations was pulverized to an average particle diameter of 1 micron by a sand grinder. Then, the dispersions were mixed in the following proportions to firm a coating solution.

| | parts |
|---|---|
| solution E | 36.0 |
| solution B | 9.2 |
| kaolin clay (50 % dispersion) | 12.0 |

The heat sensitive recording sheet was prepared as in Example 1, which will be described below.

EXAMPLES 7 to 18

| Solution A (developer dispersion) | parts |
|---|---|
| developer (see Table 2) | 6.0 |
| 10 % polyvinyl alcohol aqueous solution | 18.8 |
| water | 11.2 |
| **Solution B (dye dispersion)** | **parts** |
| 3-N-n-dibutylamino-6-methyl-7-anilino-fluoran | 2.0 |
| 10 % polyvinyl alcohol aqueous solution | 4.6 |
| water | 2.6 |
| **Solution C (sensitizer dispersion)** | **parts** |
| sensitizer (see Table 2) | 4.0 |
| 10 % polyvinyl alcohol aqueous solution | 5.0 |
| water | 3.0 |

Each of the dispersions having the above formulations was pulverized to an average particle diameter of 1 micron by a sand grinder. The dispersions were then mixed in the following proportions to form a coating solution.

| | parts |
|---|---|
| solution A | 36.0 |
| solution B | 9.2 |
| solution C | 12.0 |
| kaolin clay (50 % dispersion) | 12.0 |

The heat sensitive recording sheet was prepared as in Example 1, which will be described below.

EXAMPLES 19 to 45

| Solution C (developer dispersion) | parts |
|---|---|
| developer (see Table 3) | 6.0 |
| 10 % polyvinyl alcohol aqueous solution | 18.8 |
| water | 11.2 |
| **Solution B (dye dispersion)** | **parts** |
| 3-N-n-dibutylamino-6-methyl-7-anilino-fluoran | 2.0 |
| 10 % polyvinyl alcohol aqueous solution | 4.6 |
| water | 2.6 |

Each of the dispersions having the formulations was pulverized to an average particle diameter of 1 micron by a sand grinder. Then the dispersions were mixed in the following proportins to form a coating solution.

| | parts |
|---|---|
| solution C | 36.0 |
| solution B | 9.2 |
| kaolin clay (50 % dispersion) | 12.0 |

The coating solution was coated on one side of a base sheet (50 g/m$^2$) such that the coating amount reached 6.0 g/m$^2$, and dried. The sheet was treated by a supercalender such that smoothness was 500 to 600 seconds to prepare a heat sensitive recording sheet.

EXAMPLES 46 to 72

| Solution C (developer dispersion) | parts |
|---|---|
| developer (see Table 4) | 6.0 |
| 10 % polyvinyl alcohol aqueous solution | 18.8 |
| water | 11.2 |
| **Solution B (dye dispersion)** | **parts** |
| 3-N-n-dibutylamino-6-methyl-7-anilino-fluoran | 2.0 |
| 10 % polyvinyl alcohol aqueous solution | 4.6 |
| water | 2.6 |

| Solution D (sensitizer dispersion) | parts |
|---|---|
| sensitizer (see Table 4) | 4.0 |
| 10 % polyvinyl alcohol aqueous solution | 5.0 |
| water | 3.0 |

Each of the dispersions having the above formulations was pulverized to an average particle diameter of 1 micron by a sand grinder. Then the dispersions were mixed in the following proportions to form a coating solution.

| | parts |
|---|---|
| solution C | 36.0 |
| solution B | 9.2 |
| solution D | 12.0 |
| kaolin clay (50 % dispersion) | 12.0 |

The heat sensitive recording sheet was prepared as in Example 1, which will be described below.

By the way, the properties of the heat sensitive recording sheets obtained in said Examples and Comparative Examples were measured by the belowdescribed methods. The results are shown in Tables 2, 3 and 4.

(1) Static color concentration:

A heat sensitive recording sheet was pressed against a hot plate heated at 105°C and a pressure of 10 g/cm$^2$ for 5 seconds, and the color concentration of the sheet was measured by a Macbeth densitometer (RD-914, using an amber filter).

(2) Dynamic color concentration:

Using a heat sensitive facsimile UF-1000 manufactured by Matsushita Denso K.K., an image concentration recorded with an impression energy of 0.58 mj/Dot and a pulse width of 0.97 millisecond was measured by the Macbeth densitometer.

(3) Discoloration with oil ink:

The sample of the heat sensitive recording sheet was written with Red Magic Ink No. 500 made by Teranishi Kagaku K.K., and a degree of discoloration to the inherent red color was observed with an unaided eye, and graded as follows.

◎ ... not discolored
○ ... little discolored
△ ... slightly discolored
X ... heavily discolored

Table 2

Performance test results

| | | Developer | Sensitizer | Color concentration | | Discoloration by oil ink (3) |
|---|---|---|---|---|---|---|
| | | | | static (1) | dynamic (2) | |
| Example | 1 | 4-hydroxy-3-methyl-3'-methyl-4'-methoxydiphenylsulfone | - | 0.70 | 0.65 | ○ |
| | 2 | 4-hydroxy-3-methyl-4'-methoxydiphenylsulfone | - | 0.68 | 0.62 | ○ |
| | 3 | 4-hydroxy-3-methyl-4'-ethoxy-diphenylsulfone | - | 0.65 | 0.61 | ○ |
| | 4 | 4-hydroxy-3'-methyl-4'-ethoxy diphenylsulfone | - | 0.69 | 0.63 | ○ |
| | 5 | 4-hydroxy-3-methyl-3'-methyl-4'-ethoxydiphenylsulfone | - | 0.71 | 0.64 | ○ |
| | 6 | 4-hydroxy-3-methyl-3'-methyl-4'-propoxydiphenylsulfone | - | 0.69 | 0.61 | ○ |
| Comparative Example | 1 | 4-hydroxy-4'-methoxy-diphenylsulfone | - | 0.65 | 0.35 | X |
| | 2 | 4-hydroxy-4'-ethoxydiphenyl-sulfone | - | 0.40 | 0.34 | X |
| | 3 | 4-hydroxy-4'-propoxydiphenyl-sulfone | - | 0.70 | 0.70 | X |
| | 4 | 4-hydroxy-4'-isopropoxy-diphenylsulfone | - | 0.80 | 0.75 | X |

- continued -

Table 2

Performance test results

| | | Developer | Sensitizer | Color concentration | | Discoloration by oil ink (3) |
|---|---|---|---|---|---|---|
| | | | | static (1) | dynamic (2) | |
| Example | 7 | 4-hydroxy-3-methyl-3'-methyl-4'-methoxydiphenylsulfone | 1,2-di(3-methylphenoxy)ethane | 1.16 | 1.00 | ◎ |
| | 8 | 4-hydroxy-3-methyl-4'-methoxydiphenylsulfone | di(p-methylbenzyl) oxalate | 1.16 | 1.01 | ◎ |
| | 9 | 4-hydroxy-3-methyl-4'-ethoxydiphenylsulfone | p-benzylphenyl | 1.15 | 1.00 | ◎ |
| | 10 | 4-hydroxy-3'-methyl-4'-ethoxydiphenylsulfone | ß-benzyloxynaphthalene | 1.14 | 0.99 | ◎ |
| | 11 | 4-hydroxy-3-methyl-3'-methyl-4'-ethoxydiphenylsulfone | 4-biphenyl-p-tolyl ether | 1.16 | 1.01 | ◎ |
| | 12 | 4-hydroxy-3-methyl-3'-methyl-4'-propoxydiphenylsulfone | 4-biphenyl-p-tolyl ether | 1.16 | 1.02 | ◎ |
| | 13 | 4-hydroxy-3-methyl-3'-methyl-4'-methoxydiphenylsulfone | diphenylcarbonate | 0.79 | 0.70 | ○ |
| | 14 | 4-hydroxy-3-methyl-4'-methoxydiphenylsulfone | phenyl p-toluenesulbonate | 0.82 | 0.71 | ○ |
| | 15 | 4-hydroxy-3-methyl-4'-ethoxydiphenylsulfone | dimethyl terephthalate | 0.75 | 0.73 | ○ |

- continued -

Table 2

Performance test results

| Example | | Developer | Sensitizer | Color concentration static (1) | Color concentration dynamic (2) | Discoloration by oil ink (3) |
|---|---|---|---|---|---|---|
| Example | 16 | 4-hydroxy-3'-methyl-4'-ethoxydiphenylsulfone | dibenzyl terephthalate | 0.80 | 0.69 | ○ |
| | 17 | 4-hydroxy-3-methyl-3'-methyl-4'-ethoxydiphenylsulfone | phenyl 1-hydroxy-2-naphthoate | 0.77 | 0.72 | ○ |
| | 18 | 4-hydroxy-3-methyl-3'-methyl-4'-propoxydiphenylsulfone | methyl p-benzylbenzoate | 0.80 | 0.74 | ○ |

Table 3

Performance test results

| | | Developer | Color concentration | | Discoloration by oil ink (3) |
|---|---|---|---|---|---|
| | | | static (1) | dynamic (2) | |
| Example | 19 | 4-hydroxy-2'-phenyl-4'-phenoxydiphenylsulfone | 0.71 | 0.64 | ○ |
| | 20 | 4-hydroxy-3-p-chlorophenyl-2'-phenyl-4'-p-methoxyphenoxydiphenylsulfone | 0.66 | 0.63 | ○ |
| | 21 | 4-hydroxy-3-cyclohexyl-4'-(4-chloro-cyclohexyloxy)diphenylsulfone | 0.64 | 0.61 | ○ |
| | 22 | 4-hydroxy-2,5-dicyclohexyl-4'-benzyloxydiphenylsulfone | 0.68 | 0.62 | ○ |
| | 23 | 4-hydroxy-3-ethyl-4'-vinyloxydiphenyl-sulfone | 0.70 | 0.63 | ○ |
| | 24 | 4-hydroxy-3-propyl-2'-propyl-4'-p-methoxyphenoxydiphenylsulfone | 0.68 | 0.60 | ○ |
| | 25 | 4-hydroxy-3-propyl-3'-propyl-4'-cyclohexyloxydiphenylsulfone | 0.65 | 0.63 | ○ |
| | 26 | 4-hydroxy-3-ethyl-4'-methoxydiphenyl-sulfone | 0.70 | 0.64 | ○ |
| | 27 | 4-hydroxy-2'-ethyl-4'-methoxydiphenyl-sulfone | 0.71 | 0.62 | ○ |

- continued -

Table 3

Performance test results

| | | Developer | Color concentration | | Discoloration by oil ink (3) |
|---|---|---|---|---|---|
| | | | static (1) | dynamic (2) | |
| Example | 28 | 4-hydroxy-3-propyl-2'-propyl-4'-propoxydiphenylsulfone | 0.66 | 0.63 | ○ |
| | 29 | 4-hydroxy-3-propyl-3'-propyl-4-propoxydiphenylsulfone | 0.67 | 0.60 | ○ |
| | 30 | 4-hydroxy-3-methyl-4'-propoxydiphenylsulfone | 0.68 | 0.60 | ○ |
| | 31 | 4-hydroxy-3-methyl-3'-methyl-4'-propoxydiphenylsulfone | 0.69 | 0.61 | ○ |
| | 32 | 4-hydroxy-3-methyl-3'-methyl-4'-pentyloxydiphenylsulfone | 0.70 | 0.60 | ○ |
| | 33 | 4-hydroxy-3-methyl-3'-methyl-4'-cyclohexyloxydiphenylsulfone | 0.71 | 0.65 | ○ |
| | 34 | 4-hydroxy-3-methyl-3'-methyl-4'-benzyloxydiphenylsulfone | 0.68 | 0.63 | ○ |
| | 35 | 4-hydroxy-3-methyl-3'-methyl-4'-m-methylbenzyloxydiphenylsulfone | 0.66 | 0.62 | ○ |
| | 36 | 4-hydroxy-3-methyl-3'-methyl-4'-butoxydiphenylsulfone | 0.71 | 0.60 | ○ |

- continued -

Table 3

Performance test results

| | | Developer | Color concentration static (1) | Color concentration dynamic (2) | Discoloration by oil ink (3) |
|---|---|---|---|---|---|
| Example | 37 | 4-hydroxy-3-methyl-3'-methyl-4'-isopropoxydiphenylsulfone | 0.70 | 0.61 | ○ |
| | 38 | 4-hydroxy-3-methyl-3'-methyl-4'-isobutoxydiphenylsulfone | 0.68 | 0.62 | ○ |
| | 39 | 4-hydroxy-2-methyl-2'-methyl-4'-methoxydiphenylsulfone | 0.70 | 0.65 | ○ |
| | 40 | 4-hydroxy-2-methyl-2'-methyl-4'-ethoxydiphenylsulfone | 0.71 | 0.66 | ○ |
| | 41 | 4-hydroxy-3-ethyl-4'-ethoxy-diphnylsulfone | 0.68 | 0.65 | ○ |
| | 42 | 4-hydroxy-3-ethyl-4'-propoxy-diphenylsulfone | 0.70 | 0.64 | ○ |
| | 43 | 4-hydroxy-3-isopropyl-4'-ethoxy-diphenylsulfone | 0.69 | 0.64 | ○ |
| | 44 | 4-hydroxy-3-isopropyl-4'-propoxy-diphenylsulfone | 0.72 | 0.62 | ○ |
| | 45 | 4-hydroxy-3-cyclohexyl-4'-ethoxy-diphenylsulfone | 0.67 | 0.60 | ○ |

Table 4

Performance test results

| Example | | Developer | Sensitizer | Color concentration | | Discoloration by oil ink (3) |
|---|---|---|---|---|---|---|
| | | | | static (1) | dynamic (2) | |
| Example | 46 | 4-hydroxy-2'-phenyl-4'-phenoxy-diphenylsulfone | 1,2-di(3-methyl-phenoxy)ethane | 1.15 | 1.00 | ◎ |
| | 47 | 4-hydroxy-3-p-chlorophenyl-2'-phenyl-4'-p-methoxyphenoxydiphenylsulfone | di(p-methyl-benzyl) oxalate | 1.16 | 1.01 | ◎ |
| | 48 | 4-hydroxy-3-cyclohexyl-4'-(4-chloro-cyclohexyloxy)diphenylsulfone | p-benzylphenyl | 1.15 | 1.00 | ◎ |
| | 49 | 4-hydroxy-2,5-dicyclohexyl-4'-benzyloxydiphenylsulfone | ß-benzyloxy-naphthalene | 1.14 | 0.99 | ◎ |
| | 50 | 4-hydroxy-3-ethyl-4'-vinyloxy-diphenylsulfone | 4-biphenyl-p-tolyl ether | 1.15 | 1.02 | ◎ |
| | 51 | 4-hydroxy-3-propyl-2'-propyl-4'-p-methoxyphenoxydiphenylsulfone | m-terpheny | 1.16 | 1.01 | ◎ |
| | 52 | 4-hydroxy-3-propyl-3'-propyl-4'-cyclohexyloxydiphenylsulfone | 1,2-diphenoxy-ethane | 1.15 | 1.00 | ◎ |
| | 53 | 4-hydroxy-3-ethyl-4'-methoxy-diphenylsulfone | dibenzyl oxalate | 1.14 | 1.01 | ◎ |
| | 54 | 4-hydroxy-2'-ethyl-4'-methoxy-diphenylsulfone | (p-chloro-benzyl) oxalate | 1.15 | 0.99 | ◎ |

- continued -

Table 4

Performance test results

| | | Developer | Sensitizer | Color concentration | | Discoloration by oil ink (3) |
|---|---|---|---|---|---|---|
| | | | | static (1) | dynamic (2) | |
| Example | 55 | 4-hydroxy-3-propyl-2'-propyl-4'-propoxydiphenylsulfone | 1,2-di(3-methylphenoxy)ethane | 1.15 | 1.01 | ◎ |
| | 56 | 4-hydroxy-3-propyl-3'-propyl-4'-propoxydiphenylsulfone | di(p-methyldibenzyl) oxalate | 1.16 | 1.00 | ◎ |
| | 57 | 4-hydroxy-3-methyl-4'-propoxydiphenylsulfone | p-benzylbiphenyl | 1.15 | 0.99 | ◎ |
| | 58 | 4-hydroxy-3-methyl-3'-methyl-4'-propoxydiphenylsulfone | ß-benzyloxynaphthalene | 1.14 | 1.00 | ◎ |
| | 59 | 4-hydroxy-3-methyl-3'-methyl-4'-pentyloxydiphenylsulfone | 4-biphenyl-p-tolyl ether | 1.16 | 0.99 | ◎ |
| | 60 | 4-hydroxy-3-methyl-3'-methyl-4'-cyclohexyloxydiphenylsulfone | m-terphenyl | 1.15 | 1.01 | ◎ |
| | 61 | 4-hydroxy-3-methyl-3'-methyl-4'-benzyloxydiphenylsulfone | 1,2-diphenoxyethane | 1.14 | 1.01 | ◎ |
| | 62 | 4-hydroxy-3-methyl-3'-methyl-4'-m-methylbenzyloxydiphenylsulfone | benzyl oxalate | 1.16 | 0.99 | ◎ |
| | 63 | 4-hydroxy-3-methyl-3'-methyl-4'-butoxydiphenylsulfone | (p-chlorobenzyl) oxalate | 1.15 | 1.01 | ◎ |

- continued -

Table 4

Performance test results

| Example | Developer | Sensitizer | Color concentration static (1) | Color concentration dynamic (2) | Discoloration by oil ink (3) |
|---|---|---|---|---|---|
| 64 | 4-hydroxy-3-methyl-3'-methyl-4'-isopropoxydiphenylsulfone | 1,2-di(3-methylphenoxy)ethane | 1.15 | 1.02 | ◎ |
| 65 | 4-hydroxy-3-methyl-3'-methyl-4'-isobutoxydiphenylsulfone | di(p-methylbenzyl) oxalate | 1.15 | 1.01 | ◎ |
| 66 | 4-hydroxy-2-methyl-2'-methyl-4'-methoxydiphenylsulfone | 1,2-di(3-methylphenoxy)ethane | 1.14 | 1.03 | ◎ |
| 67 | 4-hydroxy-2-methyl-2'-methyl-4'-ethoxydiphenylsulfone | di(p-methylbenzyl) oxalate | 1.15 | 1.02 | ◎ |
| 68 | 4-hydroxy-3-ethyl-4'-ethoxydiphenylsulfone | p-benzylbiphenyl | 1.15 | 1.01 | ◎ |
| 69 | 4-hydroxy-3-ethyl-4'-propoxydiphenylsulfone | ß-benzyloxynaphthalene | 1.16 | 1.03 | ◎ |
| 70 | 4-hydroxy-3-isopropyl-4'-ethoxydiphenylsulfone | 4-biphenyl-p-tolyl ether | 1.15 | 1.02 | ◎ |
| 71 | 4-hydroxy-3-isopropyl-4'-propoxydiphenylsulfone | m-terphenyl | 1.14 | 1.03 | ◎ |
| 72 | 4-hydroxy-3-cyclohexyl-4'-ethoxydiphenylsulfone | dibenzyl oxalate | 1.15 | 1.01 | ◎ |

## Claims

**1.** 4-Hydroxydiphenylsulfone derivatives represented by formula (I)

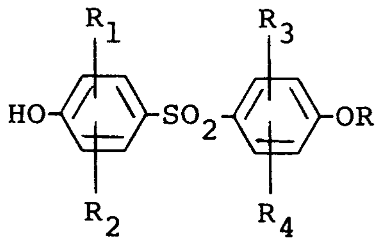

(I)

wherein
$R_1$, $R_2$, $R_3$ and $R_4$, independently from one another, each denote a hydrogen atom, an alkyl group, a substituted or unsubstituted phenyl group or a cycloalkyl group, provided at least one of $R_1$, $R_2$, $R_3$ and $R_4$ is other than a hydrogen atom, and
R denotes an alkyl group, an alkenyl group, a substituted or unsubstituted phenyl group, a substituted or unsubstituted cycloalkyl group or a substituted or unsubstituted phenylalkyl group.

**2.** The derivatives of claim 1 wherein $R_1$, $R_2$, $R_3$ and $R_4$, independently from one another, are each a hydrogen atom, a lower alkyl group, a phenyl group that may optionally be substituted by halogen, or a cyclohexyl group, provided at least one of $R_1$, $R_2$, $R_3$ and $R_4$ is other than a hydrogen atom.

**3.** The derivatives of claim 1 wherein $R_1$ and $R_3$, independently from each other, are each a hydrogen atom, a lower alkyl group, a phenyl group that may optionally be substituted by chlorine, or a cyclohexyl group, provided $R_1$ and $R_3$ are not hydrogen atoms at the same time, and $R_2$ and $R_4$ are each a hydrogen atom.

**4.** The derivatives of claim 1 wherein $R_1$ and $R_3$, independently from each other, are each a hydrogen atom or a methyl group, provided $R_1$ and $R_3$ are not hydrogen atoms at the same time.

**5.** The devivatives of claim 1 wherein R is a lower alkyl group, a $C_2$-$C_3$ alkenyl group, a phenyl group that may optionally be substituted by halogen, lower alkyl or lower alkoxy, a cyclohexyl group that may optionally be substituted by halogen, or a phenyl-lower alkyl group that may optionally be substituted by halogen, lower alkyl or lower alkoxy.

**6.** The derivatives of claim 1 wherein R is a lower alkyl group, a phenyl group that may optionally be substituted by methyl or methoxy, a cyclohexyl group that may optionally be substituted by chlorine or a benzyl group that may optionally be substituted by methyl or methoxy.

**7.** A heat sensitive recording material having on a support a heat sensitive color formation layer comprising as main components a colorless or light-colored basic leuco dye and an organic developer, characterized in that the organic developer comprises at least one of the 4-hydroxydiphenylsulfone derivatives of formula (I) recited in claim 1.

**8.** The material of claim 7 wherein the basic leuco dye is selected from triphenylmethane-type, fluoran-type and fluorene-type leuco dyes.

**9.** The material of claim 7 wherein the heat sensitive color formation layer further comprises a sensitizer.

**10.** The material of claim 9 wherein the sensitizer is at least one compound selected from the group consisting of 1,2-di(3-methylphenoxy)ethane, di(p-methylbenzyl) oxalate, p-benzylbiphenyl, betabenzyloxynaphthalene, 4-biphenyl-p-tolyl ether, m-terphenyl, 1,2-diphenoxyethane, dibenzyl oxalate and di-(p-chlorobenzyl) oxalate.

**11.** The material of claim 7 wherein the heat sensitive color formation layer contains 1 to 8 parts by weight of the organic developer per part by weight of the basic leuco dye.

**12.** The material of claim 9 wherein the sensitizer is contained in an amount of 0.1 to 5 parts by weight per part by weight of the basic leuco dye.

European Patent Office

# EUROPEAN SEARCH REPORT

Application Number

**EP 91 11 1404**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | PATENT ABSTRACTS OF JAPAN, vol. 7, no. 98 (M-210)[1243], 26th April 1983; & JP-A-58 20 493 (YOSHITOMI SEIYAKU) 05-02-1983 * The whole document * | | C 07 C 317/22<br>C 07 C 317/32<br>B 41 M 5/30 |
| A,D | GB-A-2 142 630 (SHIN NISSO KAKO CO.) | | |

TECHNICAL FIELDS SEARCHED (Int. Cl.5)

C 07 C 317/00

The present search report has been drawn up for all claims

| Place of search | Date of completion of search | Examiner |
|---|---|---|
| The Hague | 14 October 91 | VAN GEYT J.J.A. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document